# EUROPÄISCHE PATENTSCHRIFT

(11) **EP 0 337 920 B1**
(45) Veröffentlichungstag und Bekanntmachung des Hinweises auf die Patenterteilung: **16.08.1995**
(21) Anmeldenummer: 89710022.8
(22) Anmeldetag: 05.04.1989
(51) Int. Cl.: C07D 309/30, C12P 17/06

(54) **Verfahren zur hochregioselektiven Veresterung und Esterspaltung an ungesättigten Zuckerverbindungen mit Hilfe von Lipasen und Esterasen und mit diesem Verfahren herstellbare Produkte**
Process for high regioselective esterification and ether-cleavage on unsaturated sugar compounds with the help of lipases and esterases and products obtained with that process
Procédé d'estérification et de clivage d'esters hautement régiosélective appliqué à des sucres insaturés à l'aide de lipases et estérases et produits obtenus par ce procédé

(30) Priorität: 14.04.1988 DE 3812409; 19.08.1988 DE 3828190
(43) Veröffentlichungstag der Anmeldung: 18.10.1989
(73) Patentinhaber: HOECHST AKTIENGESELLSCHAFT, 65926 Frankfurt am Main (DE)
(72) Erfinder: Holla, Wolfgang, Dr., D-6238 Hofheim am Taunus (DE); Keller, Reinhold, Dr., D-6232 Soden am Taunus (DE)

(56) Entgegenhaltungen:
- CHEMICAL ABSTRACTS, Band 96, 1982, Seite 741, Zusammenfassung Nr. 181534c,Columbus, Ohio, US; A.F. HADFIELD et al.: "Cation-exchange resin-catalyzedaddition of methanol to benzoylated 1,5-anhydro-2-deoxy-D-hex-1-enitols",
- & CARBOHYDR. RES. 1982, 101(2), 197-208
- ANGEWANDTE CHEMIE INT. ED. ENGL., Band 28, Nr. 2, 1989, Seiten 220-221, VCHVerlagsgesellschaft mbH, Weinheim, DE; E.W. HOLLA: "Enzymatic synthesis ofselectively protected glycals"

## Beschreibung

Verfahren zur hochregioselektiven Veresterung und Esterspaltung an ungesättigten Zuckerverbindungen mit Hilfe von Lipasen und Esterasen und mit diesem Verfahren herstellbare Produkte

Ungesättigte Zucker finden als Synthesebausteine eine breite Anwendung, beispielsweise bei der Herstellung von Glycokonjugaten, Disacchariden, Oligosacchariden, 2-Deoxyzuckern, Aminozuckern, Thromboxanen und dem Lactonteil des Compactins.

Die für diese Synthesen notwendigen regioselektiven Transformationen bzw. Modifikationen der ungesättigten Zucker stellen in der organischen Chemie häufig ein Problem dar und erfordern schwierige bzw. aufwendige Reaktionsfolgen. Für die regioselektive chemische Acylierung der zahlreichen Hydroxylgruppen ist die zeitaufwendige Einführung und spätere Abspaltung von speziellen Schutzgruppen notwendig.

In Chemical Abstracts 96, Ref. 181534c (A.F. Hadfield, A.C. Sartorelli, Carbohydr. Res. 101, 197-208 (1982)) ist beispielsweise ein durch selektive, chemische Benzoylierung hergestelltes, ungesättigtes Zuckerderivat, das 1,5-Anhydro-3,6-di-O-benzoyl-2-deoxy-arabino-hex-1-enitol beschrieben.

Es ist bekannt, daß regioselektive Veresterungen und Esterspaltungen an primären Hydroxylgruppen einfacher gesättigter Zucker mit Hilfe von ausgewählten Lipasen durchgeführt werden können [Therisod M, Klibanov A.M., J. Am. Chem. Soc. 108, 5638, (1986); Sweers H.M., Wong C.H., J. Am. Chem. Soc. 108, 6421 (1986); Kloosterman M. et al. Tetrahedron Letters 28, 2989 (1987)]. Die von Therisod und Klibanov beschriebenen Reaktionen erfordern große Enzymüberschüsse, Reaktionszeiten von mindestens zwei Tagen, wobei die Umsätze meistens unter 50 % liegen.

Sweers und Wong beschreiben die regioselektive enzymatische Spaltung von 2,3,4,6-Tetra-0-acyl-D-hexopyranosiden zum 6-OH-Derivat. Bei den Acylresten handelt es sich jedoch um langkettige Ester.

Von Kloostermann ist die regioselektive Deacetylierung von Methyl-2,3,4,6-Tetra-O-acetyl-α-D-glucopyranosid beschrieben.

Die enzymatischen Esterspaltungen von Monosaccharidpentaacetaten führen zu komplexen Mischungen verschiedener Regioisomerer. So erhalten Shaw und Klibanov [Biotech. Bioeng. 29, 648 (1987)] Gramm-Mengen von Glucose-2,3,4,6-tetraacetat, Glucosetriacetat, und zwar eine Mischung aus 2,4,6- und 3,4,6-Triacetat, sowie Glucose-4,6-diacetat.

Es wurde nun gefunden, daß ungesättigte Zucker mit Hilfe von Lipasen und Esterasen regiospezifisch bzw. regioselektiv verestert bzw. je nach Bedingungen die Esterbindungen auch gespalten werden können. Dies ist insbesondere überraschend, da Enzyme bekanntermaßen nur mit bestimmten Substraten reagieren.

Ungesättigte Zucker stellen ein neues Substrat für Lipasen dar. Sie sind empfindlicher als gesättigte Zucker, besitzen eine andere räumliche Struktur und eine zusätzliche funktionelle Gruppe. Daher war es nicht vorauszusehen, daß diese Reaktionen überhaupt ablaufen würden und schon gar nicht, daß sie derart wirtschaftlich durchzuführen sind.

Die Erfindung betrifft somit ein Verfahren zur Herstellung von Verbindungen der allgemeinen Formel I 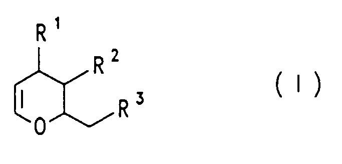
in der
- R¹: Hydroxyl und R² und R³ (C₁-C₁₀)-Acyloxy und/oder Benzoyloxy,
- R¹ und R³: (C₁-C₁₀)-Acyloxy und/oder Benzoyloxy und R² Hydroxyl oder
- R¹ und R²: Hydroxyl und R³ (C₁-C₁₀)-Acyloxy oder Benzoyloxy sind,
sowie die Hydroxyl-geschützten Derivate bedeuten, wobei die C-Atome der Acyloxygruppe mit Halogen und/oder Amin und/oder Methoxy und/oder Phenyl und/oder Phenoxy und die C-Atome der Benzoylgruppe mit Nitro und/oder Halogen und/oder (C₁ und C₂)-Alkoxy substituiert sein können, erhalten durch regioselektive bzw. regiospezifische Umsetzung mittels enzymatischer Veresterung oder Esterspaltung mit Hilfe von Lipasen und Esterasen in der R¹ und/oder R³ Position aus Verbindungen der allgemeinen Formel I in der
- R¹, R² und R³: (C₁ bis C₁₀)-Acyloxy und/oder Benzoyloxy,
- R¹, R² und R³: Hydroxyl,
- R¹: ein geschütztes Hydroxyl und R² und R³ (C₁-C₁₀)-Acyloxy und/oder Benzoyloxy,
- R¹ und R³: (C₁-C₁₀)-Acyloxy und/oder Benzoyloxy und R² ein geschütztes Hydroxyl,
- R¹: ein geschütztes Hydroxyl und R² und R³ Hydroxyl,
- R¹ und R³: Hydroxyl und R² ein geschütztes Hydroxyl,
- R¹ und R²: Hydroxyl und R³ (C₁-C₁₀)-Acyloxy oder Benzoyloxy oder ein geschütztes Hydroxyl und
- R¹ und R²: Acyloxy und/oder Benzoyloxy und R³ ein geschütztes Hydroxyl sind, wobei die C-Atome der Acyloxygruppe mit Halogen und/oder Amino und/oder Methoxy und/oder Phenyl und/oder Phenoxy und die C-Atome der Benzoyloxygruppe mit Nitro und/oder Halogen und/oder (C₁ und C₂)Alkoxy substituiert sein können.

Ferner betrifft die Erfindung die Verbindungen der allgemeinen Formel I 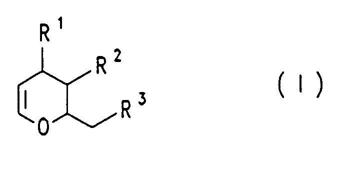
in der
i) R¹ Hydroxyl und R² und R³ (C₁-C₁₀)-Acyloxy und/oder Benzoyloxy,
j) R¹ und R³ (C₁-C₁₀)-Acyloxy und/oder Benzoyloxy und R² Hydroxyl,
k) R¹ und R² Hydroxyl und R³ (C₁-C₁₀)-Acyloxy oder Benzoyloxy sind,
sowie die Hydroxyl-geschützten Derivate, wobei die C-Atome der Acyloxygruppe mit Halogen und/oder Amin und/oder Methoxy und/oder Phenyl und/oder Phenoxy und die C-Atome der Benzoylgruppe mit Nitro und/oder Halogen und/oder C₁ und C₂)-Alkoxy substituiert sein können, ausgenommen die Verbindung der Formel I, in der R¹ und R³ Benzoyloxy und R² Hydroxyl bedeutet.

Die Erfindung betrifft auch die Verwendung dieser Verbindungen als Zwischenprodukte bei der Herstellung von Glycokonjugaten, Disacchariden, Oligosacchariden, 2-Deoxyzuckern, Aminozuckern, Thromboxanen und dem Lactonteil des Compactins .

Im folgenden wird die Erfindung detailliert beschrieben, insbesondere in ihren bevorzugten Ausführungsformen. Ferner wird die Erfindung in den Ansprüchen definiert.

Die ungesättigte Verbindung der allgemeinen Formel I ist käuflich (Glucal und Galactal bzw. deren Triacetylverbindungen 3,4,6-Tri-O-acetyl-1,5-anhydro-2-deoxy-arabino-hex-1-enitol oder 3,4,6-Tri-O-acetyl-1,5-anhydro-2-deoxy-lyxo-hex-1-enitol) oder durch chemische Synthese leicht erhältlich [Roth W., Pigman W., Methods Carbohydr. Chem. 2, 405 (1963)].

Bei den geschützten Derivaten der ungesättigten Zuckerverbindungen der allgemeinen Formel I handelt es sich, im wesentlichen a) um Acetale, bevorzugt beispielsweise Tetrahydropyranylether (THP-Ether Methoxymethylether (MOM-Ether), Methylthiomethylether (MTM-Ether), 2-Methoxyethoxymethylether (MEM-Ether), 2-(Trimethylsilyl) ethoxymethylether (SEM-Ether) und 1-Ethoxyethylether, oder b) um Ether, bevorzugt beispielsweise Methylether, Benzylether, Trimethylsilylether, tertiär-Butyldimethylsilylether, tertiär-Butyldiphenylsilylether und Allylether, sowie c) um Ester, bevorzugt beispielsweise Acetate, Chloracetate, Trifluoracetat, Benzoate, Pivaloat, Trifluormethansulfonsäureester, Methansulfonsäureester und Toluolsulfonsäureester.

Unter Acyloxy werden Gruppen mit 1 bis 10 C-Atomen, bevorzugt 1 bis 5 C-Atomen verstanden. Diese C-Atome können mit Halogen, Amino, Methoxy, Phenyl und/oder Phenoxy substituiert sein. Benzoyloxy kann mit Nitro, Halogen und/oder (C₁ und C₂)-Alkoxy substituiert sein.

Erfindungsgemäß können Lipasen und Esterasen in dem Verfahren eingesetzt werden, die vorzugsweise aus Mikroorganismen bzw. Pankreas oder Leber von Tieren gewonnen werden. Insbesondere bevorzugt werden Lipasen und Esterasen aus Pseudomonas, Candida, Mucor, Rhizopus, Penicillium und Aspergillus sowie aus Schweineleber und Schweinepankreas verwendet. Die Enzyme sind käuflich. bzw. können nach bekannten Methoden aus dem entsprechenden Mikroorganismus bzw. aus der Leber oder dem Pankreas extrahiert werden. Die Reaktion kann auch mit ganzen Zellen der genannten Mikroorganismen durchgeführt werden, soweit sie das dafür notwendige Enzym enthalten. Es ist dann unter Umständen vorteilhaft die Zellen mit Hilfe an sich bekannter Methoden durchlässig zu machen, so daß die Enzyme leichter an das Substrat gelangen können.

Bei der enzymatischen Veresterung geht man am besten wie folgt vor:
Man löst die Verbindungen Ib, e, f oder g in einem Lösungsmittel, beispielsweise Essigester, Dimethoxyethan, Tetrahydrofuran, tert. Butylmethylether oder Methylethylketon, und gibt dann den Benzoyl- oder Acyldonor hinzu, z.B. einen Carbonsäureester bevorzugt einen Vinylester, wie beispielsweise Vinylacetat, Benzoesäurevinylester, Chloressigsäurevinylester, Methoxyessigsäurevinylester, Phenoxyessigsäurevinylester, Phenylessigsäurevinylester, Aminosäurevinylester, z.B. Z-Glycinvinylester oder Isopropenylacetat, oder anstelle eines Carbonsäureesters die Carbonsäureanhydride, wie z.B. Pivalinsäureanhydrid, Essigsäureanhydrid und Benzoesäureanhydrid. Man kann aber auch den Benzoyl- oder Acyldonor gleichzeitig als Lösungsmittel benutzen. Die Lipase oder Esterase wird in freier oder immobilisierter Form zugegeben, wobei bei Temperaturen zwischen 0 und 80°C, vorzugsweise zwischen 10 und 50°C inkubiert wird.

Zur Immobilisierung der Enzyme kommen alle gängigen, in der Literatur beschriebenen Verfahren in Frage [W. Hartmeier, Trends in Biotechnology 3, 149 (1985); A Rosevaer, J. Chem. Tech. Biotechnol. 34B, 127 (1984)]. Immobilisierte und freie Enzyme können auch im Säulenverfahren eingesetzt werden.

Die Reaktionszeiten sind von Struktur und Löslichkeit des ungesättigten Zuckers, von der Temperatur und von den Konzentrationsverhältnissen, insbesondere der Enzymmenge abhängig. Sie können zwischen 0,5 Stunden und 3 Tagen liegen, jedoch in aller Regel zwischen 5 und 24 Stunden.

Die Aufarbeitung der Reaktionen erfolgt meist durch Abtrennen, z.B. Abfiltrieren, des freien oder immobilisierten Enzyms, Abdestillieren der Lösungsmittel und/oder der Acyldonoren im Vakuum und Reinigung der Produkte, sofern nötig, durch Kristallisation, Chromatographie oder Extraktion.

Zur enzymatischen Hydrolyse gibt man die Verbindungen Ia,c,d,f oder h in reiner oder gelöster Form in eine gepufferte, wäßrige Lösung und gibt das gewünschte Enzym hinzu. Man kann sowohl bei konstantem pH-Wert als auch ohne pH-Wert-Kontrolle im Bereich von etwa 4 bis 8 arbeiten. Nach Beendigung der Reaktion erhält man das gewünschte Produkt durch Extraktion oder nach Gefrier-Trocknung und Abtrennen von Enzym und Begleitsalzen durch z. B. Filtration oder Chromatographie.

Bestimmte Reaktionen werden vorteilhaft mit bestimmten Enzymen durchgeführt, insbesondere um beste Reaktionsgeschwindigkeiten und Ausbeuten zu erzielen. Die Esterspaltung der Verbindung mit der Formel Ia, in der R¹, R² und R³ Acyloxy und/oder Benzoyloxy bedeutet, wird bevorzugt mit Lipasen bzw. Esterasen aus Pseudomonaden durchgeführt. Als Reaktionsprodukt entsteht die Verbindung der Formel Ii, in der R¹ Hydroxyl und R² und R³ Acyloxy und/oder Benzoyloxy bedeutet. Die Veresterung wiederum kann mit Hilfe von Pseudomonas- bzw. Candida-Lipasen bzw. -Esterasen vorteilhaft durchgeführt werden, jedoch sind die Hauptendprodukte unterschiedlich. Aus der Verbindung der Formel Ib, in der R¹, R² und R³ Hydroxyl bedeuten, entsteht durch Inkubation mit dem Enzym aus Pseudomonas die Verbindung der Formel Ij, in der R¹ und R³ Acyloxy und/oder Benzoyloxy und R² Hydroxyl bedeuten. Die Übertragung von Benzoylgruppen auf R¹ ist jedoch langsamer als die Übertragung von Acylgruppen, höchstwahrscheinlich aus sterischen Gründen, so daß Pseudomonas-Lipasen auch zur Bildung des 6-O-Monobenzoats genutzt werden können. Mit Candida-Lipasen kann man die Verbindung der Formel Ik erhalten, in der R¹ und R² Hydroxyl bedeuten und R³ Acyloxy oder Benzoyloxy ist.

Die entstandenen Verbindungen Ii,j,k können mit Hilfe der obengenannten Schutzgruppen nach an sich bekannten Methoden chemisch derivatisiert werden (T. W. Greene, Protective Groups in Organic Synthesis, John Wiley & Sons, 1981), um sie dann entweder in organisch-chemischen Reaktionen und/oder erneut in regioselektiven Veresterungen bzw. Esterspaltungen mit Lipasen und Esterasen einzusetzen. So wird beispielsweise chemisch die Verbindung der Formel I hergestellt, in der R¹ eine geschützte Hydroxyfunktion und R² und R³ eine Acyloxygruppe darstellen, und mit Hilfe der Lipasen und Esterasen aus R³ die Acylgruppe abgespalten, so daß R³ dann Hydroxyl bedeutet. Bevorzugt wird bei diesen Reaktionen mit Candida-, Pseudomonas-, Mucor- und Pankreas-Lipasen bzw. -Esterasen gearbeitet.

Bei der Verbindung Ij kann in R² ebenfalls eine Schutzgruppe eingeführt werden. Wenn derartige Verbindungen dann vorzugsweise mit dem Enzym aus Pseudomonas oder Candida behandelt werden entstehen 2 verschiedene Produkte. Mit Hilfe der ersteren erhält man die Verbindung der Formel I, in der R¹ Hydroxyl, R² eine geschützte Hydroxylgruppe und R³ Acyloxy ist, und mit Hilfe der Candida-Lipase die Verbindung der Formel I, in der R¹ Acyloxy, R² eine geschützte Hydroxylgruppe und R³ eine Hydroxylgruppe bedeutet.

Aufgrund der vorangegangenen Beobachtungen ist überraschend abzuleiten, daß Pseudomonas-Lipasen bzw. -Esterasen bevorzugt die sekundäre funktionelle Gruppe R¹ in Verbindungen der Formel I angreifen, während Candida-Lipasen bzw. -Esterasen vorzugsweise die primäre funktionelle Gruppe R³ angreifen. Bei enzymatischen Veresterungen lassen sich anstelle von Pseudomonas-Lipasen bzw. -Esterasen gegebenenfalls auch Lipasen oder Esterasen aus Mucor, Rhizopus und Penicillium spezies zur Katalyse des Angriffs an R¹ in Verbindungen der Formel I b,f,g,i einsetzen, für Hydrolysen an R¹ in Verbindungen der Formel I a,d,h,j in Einzelfällen außer den genannten auch Lipasen aus Schweinepankreas.

Die Vorteile des erfindungsgemäßen Verfahrens liegen in der unerwarteten Regioorientierung der hohen Regioselektivität und der damit verbundenen Einheitlichkeit des Produkts, der hohen Reaktionsgeschwindigkeit sowie der hohen Ausbeute und der leichten Aufarbeitung.

Anhand der folgenden Beispiele wird die Erfindung weiter detailliert beschrieben. Prozentangaben beziehen sich soweit nicht anders angegeben auf das Gewicht.

### Beispiele

### 1. Herstellung von 6-O-Acetylglucal

1,022 g (7 mmol) Glucal werden in 2 ml Essigester und 30 ml Vinylacetat aufgenommen, mit 400 mg Lipase aus Candida cylindracea Lipase-OF (Meito Sangyo Co. Ltd.) versetzt und bei Zimmertemperatur über Nacht gerührt. Nach Abtrennen des wiederverwendbaren Enzyms und anschließende Chromatographie oder Kristallisation erhält man 1,12-1,25 g 6-O-Acetylglucal (85-95 %ige Ausbeute).

### 2. Herstellung von 6-O-Acetyl-3-O-tert.-butyldimethylsilyl-glucal

1,09 g (5 mmol) 3-O-tert.-Butyldimethylsilyl-glucal werden in etwa 10 ml Essigester und 50-70 ml Vinylacetat aufgenommen und mit 1 g Lipase aus Candida cylindracea (Sigma) versetzt. Nach etwa 20-24 h stündigen Rühren bei Zimmertemperatur wird das wiederverwendbare Enzym abfiltert.
Einengen im Vakuum und anschließende einfache Säulenfiltration über Kieselgel (in Hexan bzw. Ether/Hexan v:v) ergibt 1,0 - 1,2 g des gewünschten 6-O-Acetyl-3-O-tert.-butyldimethylsilyl-glucal (80-90 %ige Ausbeute).

### 3. Herstellung von 6-O-Acetyl-4-O-tert.-butyldimethylsilyl-glucal

1,09 g (5 mmol) 4-O-tert.-Butyldimethylsilyl-glucal werden
in 10-20 ml Essigester und 50-80 ml Vinylacetat aufgenommen und mit 1,0 g Lipase aus Candida cylindracea (Sigma) versetzt. Nach 20-24 stündigem Rühren bei Zimmertemperatur wird das wiederverwendbare Enzym abfiltriert und die Lösung im Vakuum eingeeengt. Anschließende einfache Säulenfiltration über Kieselgel in Ether/Hexan (∼ 1:1 v:v) ergibt 1,1 - 1,2 g 6-O-Acetyl-4-O-tert.-butyldimethylsilyl-glucal (85-90 %ige Ausbeute.

### 4. Herstellung von 6-O-Acetylgalactal

1,0 g (6,85 mmol) Galactal wird in etwa 1 ml Wasser gelöst, mit 1 - 4 g zerriebenem Molsieb, 25 - 75 ml Vinylacetat und 800 mg Lipase aus Candida cylindracea (Sigma) versetzt. Es wird ca. 45 min. bei Zimmertemperatur gerührt. Trocknen, Filtrieren, Einengen im Vakuum und Chromatographie an Kieselgel (Essigester/Hexan 1:1) oder Kristallisation (aus Essigester/Hexan) ergibt 1,090 bis 1,160 (85-90 %) des gewünschten 6-O-Acetylgalactals.

### 5. Herstellung von 4,6-Di-O-acetylglucal

7,0 g (30 mmol) Tri-O-acetylglucal werden in 70 ml 0,25 M Phosphatpuffer (pH = 7) mit 7 g Lipase aus Pseudomonas spec. (Lipase P, Fa. Amano Pharmaceutical Co., Ltd., Nagoya, Japan) (frei oder an SiO₂ fixiert) bei Zimmertemperatur gerührt. Nach Beendigung der Reaktion (ca. 5-7 h) trennt man das wiederverwendbare Enzym ab. Man erhält das gewünschte 4,6-Di-O-acetylglucal entweder durch Extraktion der wäßrigen Lösung mit CHCl₃ oder Essigester oder nach Gefriertrocknung, anschließendem Aufnehmen in organischen Lösungsmitteln wie Essigester und Abfiltrieren der unlöslichen Begleitstoffe. Das in etwa 90 %iger Ausbeute anfallende 4,6-Di-O-acetylglucal kann ohne weitere Reinigung für weitere Umsetzungen verwendet werden.

### 6. Herstellung von 3,6-Di-O-acetylglucal

7,3 g (50 mmol) Glucal werden in 50 ml Essigester und 150 ml Vinylacetat aufgenommen und bei Zimmertemperatur mit 2 g Lipase aus Pseudomonas spec. (Lipase P, Fa. Amano Pharmaceutical Co., Ltd., Nagoya, Japan) gerührt. Nach Beendigung der Reaktion (DC-Kontrolle, ca. 48 h) wird das wiederverwendbare Enzym abfiltriert und das Lösungsmittel verdampft. Das in über 90 %iger Ausbeute anfallende 3,6-Di-O-acetylglucal kann ohne weitere Reinigung für synthetische Zwecke eingesetzt werden.

### 7. Herstellung von 3,6-Di-O-acetylgalactal

1,0 g (6,85 mmol) Galactal wird in etwa 1 ml Wasser gelöst, mit 1 - 4 g zerriebenem Molsieb, 25 - 75 ml Vinylacetat und 1,0 g Lipase aus Pseudomonas spec. (Amano) versetzt und über Nacht bei Zimmertemperatur gerührt. Nach Trocknen, Filtrieren und Chromatographie an Kieselgel (Essigester/Hexan 2:3, v:v) erhält man 1,1 bis 1,26 g (70-80 %) 3,6-Di-O-acetylgalactal.

### 8. Herstellung von 3,6-Di-O-acetylglucal

1,02 g (7 mmol) Glucal, 0,5 - 0,7 ml H₂O und etwa 2 g zerriebenes Molsieb werden in 25 - 50 ml Isopropenylacetat aufgenommen und mit 1 g Lipase aus Pseudomonas spec. versetzt und 25 - 30 h bei Zimmertemperatur gerührt. Nach Filtration und Einengen im Vakuum wird an Kieselgel chromatographiert (Essigsäureethylester/Hexan 2:3, v:v). Man erhält 0,97 - 1,05 g (60-65 %) 3,6-Di-O-acetylglucal.

### 9. Herstellung von 3-O-Acetyl-6-O-tert.butyldimethylsilylglucal

1,0 g (4 mmol) 6-O-tert.Butyldimethylsilyl-glucal wird in 5-10 ml Vinylacetat 3-4 Stunden mit 1,0 g Lipase aus Pseudomonas spec. bei Raumtemperatur gerührt. Filtrieren, Einengen und Chromatographie (SiO₂, Ether/Hexan 1:3) ergibt das gewünschte 3-O-Acetyl-6-O-tert.butyldimethylsilylglucal in etwa 85 %iger Ausbeute (0,98-1,0 g).

### 10. Herstellung von 4-O-Acetyl-6-O-tert.butyldimethylsilyl-glucal

1,03 g (3 mmol) 3,4-Di-O-Acetyl-6-O-tert. butyldimethylsilyl-glucal werden in 10 ml 0,1 M Kaliumphosphatpuffer (pH = 7) mit 0,5-1,0 g Lipase P oder Lipase Fp (Amano) an SiO₂ fixiert bei Raumtemperatur gerührt. Nach Beendigung der Reaktion (5-8 h) trennt man das fixierte, wiederverwendbare Enzym ab, Man erhält das gewünschte 4-O-Acetyl-6-O-tert.butyldimethylsilyl-glucal entweder durch Extraktion der wäßrigen Lösung mit Chloroform oder Essigester oder nach Gefriertrocknung und abschließender Chromatographie an Kieselgel (Ether/Hexan 1:2) in 82 %iger Ausbeute.

### 11. Herstellung von 3-O-Acetyl-6-O-benzoylglucal

1,0 g (4 mmol) 6-O-Benzoylglucal wird in 10-20 ml Vinylacetat aufgenommen und mit 1 g Lipase (Pseudomonas spec.) 5 h bei Raumtemperatur gerührt. Abfiltrieren des Enzyms und Kristallisation bzw. Chromatographie an Kieselgel (Essigester/Hexan 1:1) ergibt 3-O-Acetyl-6-O-benzoylglucal in 88-94 %iger Ausbeute (1,03-1,10 g).

### 12. Herstellung von 3-O-Acetyl-6-O-benzoylgalactal

1,0 g (4 mmol) 6-O-Benzoylgalactal wird in 10-15 ml Dimethoxyethan (DME) und 20-25 ml Vinylacetat aufgenommen und mit 1 g Lipase P 8 h bei Raumtemperatur gerührt. Abfiltrieren des Enzyms und Kristallisation bzw. Chromatographie an Kieselgel (Ether/Hexan 1:1) ergibt 3-O-Acetyl-6-O-Benzoylgalactal in 80-85 % Ausbeute.

### 13. Herstellung von 6-O-Acetyl-3-O-chloracetyl-glucal

1,03 g (5,5 mmol) 6-O-Acetylglucal werden in etwa 2-5 ml Dimethoxyethan und 10 ml Chloressigsäurevinylester aufgenommen und ca. 2-3 h bei Raumtemperatur mit 1 g Lipase P (Amano) gerührt. Nach Abfiltrieren des Enzyms, Einengen der Lösung und Chromatographie an Kieselgel (Ether/Hexan 1:2) erhält man 6-O-Acetyl-3-O-chloracetyl-glucal in 80-85 % Ausbeute.

### 14. Herstellung von 6-O-Acetyl-3-O-chloracetyl-galactal

1,03 g (5,5 mmol) 6-O-Acetylgalactal werden in 10-15 ml DME und 20-25 ml Chloressigsäurevinylester aufgenommen und 5-6 h bei Raumtemperatur mit 1 g Lipase P (Pseudomonas spec.) gerührt. Nach Abfiltrieren des wieder verwendbaren Enzyms, Einengen der Lösung und Chromatographie (SiO₂, Ether/Hexan 1:2) erhält man 6-O-Acetyl-3-0-chloracetyl-galactal in 80 % Ausbeute.

### 15. Herstellung von 6-O-Benzoyl-3-O-chloracetyl-glucal

1,0 g (4 mmol) 6-O-Benzoylglucal wird in 2-5 ml Dimethoxyethan und 10-15 ml Chloressigsäurevinylester gelöst und mit 1 g Lipase P (Amano) 1-2 h gerührt. Abfiltrieren des Enzyms und Chromatographie an Kieselgel (Ether/Hexan 1:1) ergibt 6-O-Benzoyl-3-O-chloracetyl-glucal in 82-87 %iger Ausbeute.

### 16. Herstellung von 6-O-Benzoyl-3-O-chloracetyl-galactal

1,0 g (4 mmol) 6-O-Benzoylglactal wird in etwa 10-15 ml DME und 20-25 ml Chloressigsäurevinylester aufgenommen und ca. 5-7 h bei Raumtemperatur mit 1 g Lipase aus Pseudomonas spec. (Lipase P, Amano) gerührt. Nach Abfiltrieren des wieder verwendbaren Enzyms, Einengen der Lösung im Vakuum und Chromatographie an Kieselgel (Ether/Hexan 1:1) bzw. Kristallisation erhält man 6-O-Benzoyl-3-O-chloracetyl-galactal in 80 % Ausbeute (1050 mg).

### 17. Herstellung von 6-O-Benzoylglucal

1,0 g (6,85 mmol) Glucal wird in 1,5-2,0 ml Tetrahydrofuran und 3-5 ml Benzoesäurevinylester aufgenommen und 6 h mit 1 g Lipase aus Candida cylindracea (Amano AY-20) bei Raumtemperatur gerührt. Nach Abfiltrieren des Enzyms, Einengen der Lösung im Vakuum, Extraktion des in Essigester aufgenommenen Rückstandes mit wäßriger NaHCO₃-Lösung und anschließende Chromatographie an Kieselgel (Essigester/Hexan 1:1) erhält man 1,20 g (70 %) des gewünschten 6-O-Benzoylglucals.

### 18. Herstellung von 6-O-Benzoylgalactal

1,0 g (6,85 mmol) Galactal wird in 0,5-1,5 ml H₂O aufgenommen, mit 8 g zeriebenem Molsieb versetzt und 8-10 h bei Raumtemperatur mit 1g Candida-Lipase AY-20 (Meito, Sangyo) oder 1 g Lipase P (Amano) in 10-15 ml Benzoesäurevinylester gerührt. Nach Abfiltrieren des Enzyms, Einengen der Lösung im Vakuum, Extraktion des in Essigester aufgenommenen Rückstandes mit wäßriger NaHCO₃-Lösung und anschließende Chromatographie an Kieselgel (Essigester/Hexan 1:1) erhält man 65-68 % (ca. 1130 mg) des gewünschten 6-O-Benzoylgalactals.

### 19. Herstellung von 6-O-Acetyl-3-O-methoxyacetylglucal

325 mg (1,73 mmol) 6-O-Acetylglucal werden in 1 ml Dimethoxyethan gelöst, mit 1 ml Methoxyessigsäurevinylester und 325 mg Lipase Fp (Pseudomonas fluorescens, Amano) versetzt und 5 ½ Stunden bei Raumtemperatur gerührt. Nach Abfiltrieren des Enzyms und Flash-Chromatographie an Kieselgel (Essigester/Hexan 1:2) erhält man 422 mg (1,62 mmol, 93,8 % Ausbeute) des gewünschten 3-O-Methoxyacetyl-6-O-Acetylglucals.

### 20. Herstellung von 6-O-Benzoyl-3-O-methoxyacetylglucal

200 mg (0,8 mmol) 6-O-Benzoylglucal werden 3 h bei Raumtemperatur in 1 ml Dimethoxyethan mit 1 ml Methoxyessigsäurevinylester und 200 mg Lipase Fp aus Pseud. fluorescens gerührt.

Abfiltrieren des Enzyms und Flash-Chromatographie an Kieselgel (Hexan und Ether/Hexan 1:1) ergeben 232 mg (0,72 mmol, 90 % Ausbeute) des gewünschten 6-O-Benzoyl-3-O-methoxyacetylglucal.

### 21. Herstellung von 6-O-Acetyl-3-O-phenoxyacetylglucal

318 mg (1,69 mmol) 6-O-Acetylglucal werden 7 Stunden in 1 ml Dimethoxyethan mit 1 ml Phenoxyessigsäurevinylester und 320 mg Lipase Fp bei Raumtemperatur gerührt.

Abfiltrieren des Enzyms und Flash-Chromatographie an Kieselgel (Essigester/Hexan 1:2) ergaben 478 mg (1,48 mmol, 87,8 % Ausbeute) 6-O-Acetyl-3-O-phenoxyacetylglucal.

### 22. Herstellung von 6-O-Benzoyl-3-O-phenoxyacetylglucal

226 mg (0,90 mmol) 6-O-Benzoylglucal werden in 1 ml Dimethoxyethan gelöst und mit 1 ml Phenoxyessigsäurevinylester und 220 mg Lipase Fp 3 Stunden bei Raumtemperatur gerührt. Nach Filtration und Flash-Chromatographie (Kieselgel; Hexan und Ether/Hexan 1:1) erhält man 6-O-Benzoyl-3-O-phenoxyacetylglucal in 88 %iger Ausbeute (304,5 mg, 0,79 mmol).

### 23. Herstellung von 6-O-Benzoyl-3-O-phenacetylglucal

200 mg (0,80 mmol) 6-O-Benzoylglucal werden in 1 ml Dimethoxyethan gelöst und mit 1 ml Phenylessigsäurevinylester und 200 mg Lipase Fp aus Pseudomonas fluorescens über Nacht (< 20 h) bei Raumtemperatur gerührt. Nach Abfiltrieren des Enzyms und chromatographischer Reinigung an SiO₂ (Hexan und Ether/Hexan 1:1) erhält man 6-O-Benzoyl-3-O-phenacetylglucal in 75-85 %iger Ausbeute (220-250 mg).

### 24. Herstellung von 6-O-Acetyl-3-O-phenacetylglucal

214 mg (1,14 mmol) 6-O-Acetylglucal werden in 1 ml Dimethoxyethan gelöst, mit 1 ml Phenylessigsäurevinylester und 200 mg Lipase Fp versetzt und 48 h bei etwa 20°C gerührt. Nach Filtration und Chromatographie erhält man 6-O-Acetyl-3-O-phenacetylglucal in 80-85 %iger Ausbeute (280-297 mg, 0,91-0,97 mmol).

## Patentansprüche

1. Verfahren zur Herstellung von Verbindungen der allgemeinen Formel I 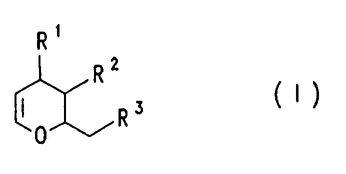 in der
R¹ Hydroxyl und R² und R³ (C₁-C₁₀)-Acyloxy und/oder Benzoyloxy,
R¹ und R³ (C₁-C₁₀)-Acyloxy und/oder Benzoyloxy und R² Hydroxyl oder
R¹ und R² Hydroxyl und R³ (C₁-C₁₀)-Acyloxy oder Benzoyloxy sind,
sowie die Hydroxyl-geschützten Derivate bedeuten, wobei die C-Atome der Acyloxygruppe mit Halogen und/oder Amin und/oder Methoxy und/oder Phenyl und/oder Phenoxy und die C-Atome der Benzoylgruppe mit Nitro und/oder Halogen und/oder (C₁ und C₂)-Alkoxy substituiert sein können, erhalten durch regioselektive bzw. regiospezifische Umsetzung mittels enzymatischer Veresterung oder Esterspaltung mit Hilfe von Lipasen und Esterasen in der R¹ und/oder R³ Position aus Verbindungen der allgemeinen Formel I in der
R¹, R² und R³ (C₁ bis C₁₀)-Acyloxy und/oder Benzoyloxy,
R¹, R² und R³ Hydroxyl,
R¹ ein geschütztes Hydroxyl und R² und R³ (C₁-C₁₀)-Acyloxy und/oder Benzoyloxy,
R¹ und R³ (C₁-C₁₀)-Acyloxy und/oder Benzoyloxy und R² ein geschütztes Hydroxyl,
R¹ ein geschütztes Hydroxyl und R² und R³ Hydroxyl,
R¹ und R³ Hydroxyl und R² ein geschütztes Hydroxyl,
R¹ und R² Hydroxyl und R³ (C₁-C₁₀)-Acyloxy oder Benzoyloxy oder ein geschütztes Hydroxyl und
R¹ und R² Acyloxy und/oder Benzoyloxy und R³ ein geschütztes Hydroxyl sind, wobei die C-Atome der Acyloxygruppe mit Halogen und/oder Amino und/oder Methoxy und/oder Phenyl und/oder Phenoxy und die C-Atome der Benzoyloxygruppe mit Nitro und/oder Halogen und/oder (C₁ und C₂)Alkoxy substituiert sein können.

2. Verfahren nach Anspruch 1, dadurch gekennzeichnet, daß die Acyloxygruppe eine Kettenlänge von C₁ bis C₅ hat.

3. Verfahren nach Anspruch 1 oder 2, dadurch gekennzeichnet, daß die Hydroxylgruppe durch eine Ester- oder eine Etherbindung geschützt oder in ein Acetal überführt wird.

4. Verfahren nach einem oder mehreren der Ansprüche 1 bis 3, dadurch gekennzeichnet, daß Lipasen und Esterbasen aus Mikroorganismen bzw. aus Pankreas oder Leber von Tieren eingesetzt werden.

5. Verfahren nach Anspruch 5, dadurch gekennzeichnet, daß Lipasen und Esterasen aus Pseudomonas, Candida, Mucor, Rhizopus, Penicillium und Aspergillus eingesetzt werden sowie aus Schweineleber und Schweinepankreas.

6. Verfahren nach Anspruch 1, dadurch gekennzeichnet, daß zur Reaktion mit primären funktionellen Gruppen der Verbindung der allgemeinen Formel I Lipasen oder Esterasen aus Candida und zur Reaktion mit sekundären funktionellen Gruppen der genannten Verbindung Lipasen oder Esterasen aus Pseudomonas eingesetzt werden.

7. Die Verbindung der allgemeinen Formel I, 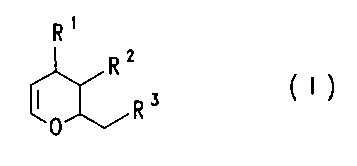 in der
i) R¹ Hydroxyl und R² und R³ (C₁-C₁₀)-Acyloxy und/oder Benzoyloxy,
j) R¹ und R³ (C₁-C₁₀)-Acyloxy und/oder Benzoyloxy und R² Hydroxyl,
k) R¹ und R² Hydroxyl und R³ (C₁-C₁₀)-Acyloxy oder Benzoyloxy sind,
sowie die Hydroxyl-geschützten Derivate, wobei die C-Atome der Acyloxygruppe mit Halogen und/oder Amin und/oder Methoxy und/oder Phenyl und/oder Phenoxy und die C-Atome der Benzoylgruppe mit Nitro und/oder Halogen und/oder C₁ und C₂)-Alkoxy substituiert sein können, ausgenommen die Verbindung der Formel I, in der R¹ und R³ Benzoyloxy und R² Hydroxyl bedeutet.

8. Verwendung der Verbindung gemäß Anspruch 7 als Zwischenprodukt bei der Herstellung von Glycokonjugaten, Disacchariden, Oligosacchariden, 2-Deoxyzuckern, Aminozuckern, Thromboxanen und dem Lactonteil des Compactins.

## Claims

1. A process for preparing compounds of the formula I 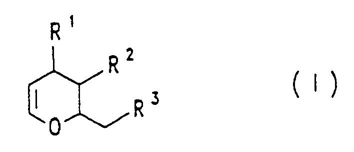 in which
R¹ is hydroxyl and R² and R³ are (C₁-C₁₀)-acyloxy and/or benzoyloxy,
R¹ and R³ are (C₁-C₁₀)-acyloxy and/or benzoyloxy and R² is hydroxyl, or
R¹ and R² are hydroxyl and R³ is (C₁-C₁₀)-acyloxy or benzoyloxy, and are also the hydroxyl-protected derivatives, where the carbon atoms of the acyloxy group can be substituted by halogen and/or amine and/or methoxy and/or phenyl and/or phenoxy, and the carbon atoms of the benzoyl group can be substituted by nitro and/or halogen and/or (C₁ and C₂)-alkoxy, obtained by regioselective or regiospecific reaction by means of enzymic esterification or ester cleavage using lipases and esterases in the R¹ and/or R³ position(s) from compounds of the formula I in which R¹, R² and R³ are (C₁ to C₁₀)-acyloxy and/or benzoyloxy,
R¹, R² and R³ are hydroxyl,
R¹ is a protected hydroxyl and R² and R³ are (C₁-C₁₀)-acyloxy and/or benzoyloxy,
R¹ and R³ are (C₁-C₁₀) -acyloxy and/or benzoyloxy and R² is a protected hydroxyl,
R¹ is a protected hydroxyl and R² and R³ are hydroxyl,
R¹ and R³ are hydroxyl and R² is a protected hydroxyl,
R¹ and R² are hydroxyl and R³ is (C₁-C₁₀)-acyloxy or benzoyloxy or a protected hydroxyl, and
R¹ and R² are acyloxy and/or benzoyloxy and R³ is a protected hydroxyl, where the carbon atoms of the acyloxy group can be substituted by halogen and/or amino and/or methoxy and/or phenyl and/or phenoxy and the carbon atoms of the benzoyloxy group can be substituted by nitro and/or halogen and/or (C₁ and C₂)-alkoxy.

2. The process as claimed in claim 1, wherein the acyloxy group has a chain length of from C₁ to C₅.

3. The process as claimed in claim 1 or 2, wherein the hydroxyl group is protected by an ester bond or an ether bond or is converted into an acetal.

4. The process as claimed in one or more of claims 1 to 3, wherein lipases and esterases are employed which derive from microorganisms or from the pancreas or liver of animals.

5. The process as claimed in claim 5, wherein lipases and esterases are employed which derive from Pseudomonas, Candida, Mucor, Rhizopus, Penicillium and Aspergillus, and also from porcine liver and porcine pancreas.

6. The process as claimed in claim 1, wherein lipases or esterases which derive from Candida are employed for the reaction with primary functional groups of the compound of the formula I and lipases or esterases which derive from Pseudomonas are employed for the reaction with secondary functional groups of the said compound.

7. A compound of the formula I, 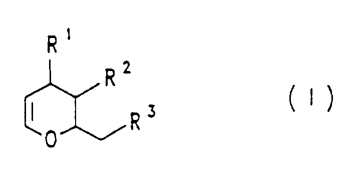 in which
i) R¹ is hydroxyl and R² and R³ are (C₁-C₁₀)-acyloxy and/or benzoyloxy,
j) R¹ and R³ are (C₁-C₁₀)-acyloxy and/or benzoyloxy and R² is hydroxyl,
k) R¹ and R² are hydroxyl and R³ is (C₁-C₁₀)-acyloxy or benzoyloxy, and also the hydroxyl-protected derivatives, where the carbon atoms of the acyloxy group can be substituted by halogen and/or amine and/or methoxy and/or phenyl and/or phenoxy, and the carbon atoms of the benzoyl group can be substituted by nitro and/or halogen and/or (C₁ and C₂)-alkoxy, with the exception of the compound of the formula I in which R¹ and R³ are benzoyloxy and R² is hydroxyl.

8. The use of a compound as claimed in claim 7 as an intermediate in the preparation of glycoconjugates, disaccharides, oligosaccharides, 2-deoxy sugars, amino sugars, thromboxanes and the lactone moeity of compactin.

## Revendications

1. Procédé pour la préparation de composés de formule générale I 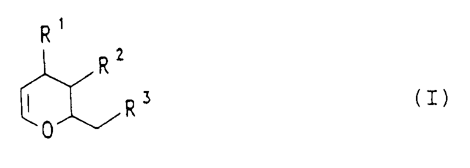 dans laquelle
R¹ est le groupe hydroxy et R² et R³ représentent un groupe acyloxy en C₁-C₁₀ et/ou le groupe benzoyloxy,
R¹ et R³ représentent un groupe acyloxy en C₁-C₁₀ et/ou le groupe benzoyloxy, et R² est le groupe hydroxy, ou
R¹ et R² représentent le groupe hydroxy et R³ représente un groupe acyloxy en C₁-C₁₀ ou benzyloxy,
ainsi que les dérivés protégés sur le groupe hydroxy, les atomes de carbone du groupe acyloxy pouvant être substitués par des halogènes et/ou par le groupe amino et/ou méthoxy et/ou phényle et/ou phénoxy, et les atomes de carbone du groupe benzoyle pouvant être substitués par le groupe nitro et/ou des halogènes et/ou des groupe alcoxy en C₁ et C₂, obtenus par une réaction régiosélective ou régiospécifique au moyen d'une estérification ou d'une coupure d'esters enzymatiques, à l'aide de lipases et d'estérases en position R¹ et/ou R³, à partir de composés de formule générale I dans lesquels
R¹, R² et R³ représentent des groupes acyloxy en C₁-C₁₀ et/ou benzoyloxy,
R¹, R² et R³ représentent le groupe hydroxy,
R¹ représente un groupe hydroxy protégé et R² et R³ représentent un groupe acyloxy en C₁-C₁₀ et/ou un groupe benzyloxy,
R¹ et R³ représentent un groupe acyloxy en C₁-C₁₀ et/ou le groupe benzoyloxy et R² représente un groupe hydroxy protégé,
R¹ représente un groupe hydroxy protégé et R² et R³ représentent le groupe hydroxy,
R¹ et R³ représentent le groupe hydroxy et R² représente un groupe hydroxy protégé,
R¹ et R² représentent le groupe hydroxy et R³ représente un groupe acyloxy en C₁-C₁₀ ou benzoyloxy, ou un groupe hydroxy protégé, et
R¹ et R² représentent un groupe acyloxy et/ou benzoyloxy, et R³ est un groupe hydroxy protégé, les atomes de carbone du groupe acyloxy pouvant être substitués par des halogènes et/ou par le groupe amino et/ou méthoxy et/ou phényle et/ou phénoxy, et les atomes de carbone du groupe benzoyloxy pouvant être substitués par le groupe nitro et/ou des halogènes et/ou des groupes alcoxy en C₁-C₂.

2. Procédé selon la revendication 1, caractérisé en ce que le groupe acyloxy a une longueur de chaîne de C₁ à C₅.

3. Procédé selon la revendication 1 ou 2, caractérisé en ce que le groupe hydroxy est protégé par une liaison ester ou éther, ou converti en un acétal.

4. Procédé selon une ou plusieurs des revendications 1 à 3, caractérisé en ce que l'on utilise des lipases et estérases provenant de micro-organismes ou de pancréas ou de foie d'animaux.

5. Procédé selon la revendication 5, caractérisé en ce que l'on utilise des lipases et estérases de *Pseudomonas*, *Candida*, *Mucor*, *Rhizopus*, *Penicillium* et *Aspergillus*, ainsi que du foie et du pancréas porcin.

6. Procédé selon la revendication 1, caractérisé en ce que dans la réaction avec des groupes fonctionnels primaires du composé de formule générale I, on utilise des lipases ou des estérases de *Candida*, et, dans la réaction avec des groupes fonctionnels secondaires dudit composé, on utilise des lipases ou des estérases de *Pseudomonas*.

7. Composé de formule générale I 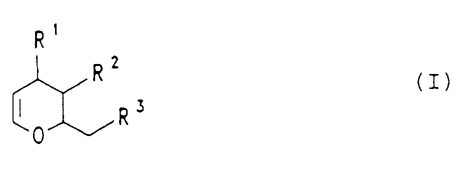 dans laquelle
i) R¹ est le groupe hydroxy et R² et R³ représentent un groupe acyloxy en C₁-C₁₀ et/ou le groupe benzoyloxy,
j) R¹ et R³ représentent un groupe acyloxy en C₁-C₁₀ et/ou le groupe benzoyloxy, et R² représente le groupe hydroxy,
k) R¹ et R² représentent le groupe hydroxy et R³ est un groupe acyloxy en C₁-C₁₀ ou benzoyloxy,
ainsi que dérivés protégés sur le groupe hydroxy, les atomes de carbone des groupes acyloxy pouvant être substitués par des halogènes et/ou par le groupe amino et/ou méthoxy et/ou phényle et/ou phénoxy, et les atomes de carbone du groupe benzoyle pouvant être substitués par le groupe nitro et/ou des halogènes et/ou des groupes alcoxy en C₁-C₂, à l'exception du composé de formule I dans lequel R¹ et R³ représentent le groupe benzoyloxy et R² représente le groupe hydroxy.

8. Utilisation du composé selon la revendication 7, en tant que produit intermédiaire dans la préparation de glycoconjugués, disaccharides, oligosaccharides, dérivés 2-désoxy de sucres, sucres aminés, thromboxanes et du fragment lactone de la compactine.
